# EUROPÄISCHE PATENTSCHRIFT

(11) **EP 1 581 481 B1**
(45) Veröffentlichungstag und Bekanntmachung des Hinweises auf die Patenterteilung: **01.10.2008**
(21) Anmeldenummer: 03782412.5
(22) Anmeldetag: 16.12.2003
(51) Int. Cl.: C07C 253/10, C07C 255/46

(54) **VERBESSERTE NEUTRALISATION VON ISOPHORONNITRIL-SYNTHESEAUSTRÄGEN**
IMPROVED NEUTRALIZATION OF ISOPHORONE NITRILE SYNTHESIS PRODUCTS
NEUTRALISATION AMELIOREE DE PRODUITS DE SYNTHESE D'ISOPHORONENITRILE

(30) Priorität: 19.12.2002 DE 10259708
(43) Veröffentlichungstag der Anmeldung: 05.10.2005
(73) Patentinhaber: BASF SE, 67056 Ludwigshafen (DE)
(72) Erfinder: OFTRING, Alfred, 67098 Bad Dürkheim (DE); BRAUN, Gerold, 67071 Ludwigshafen (DE)
(74) Vertreter: Isenbruck, Günter
(86) Internationale Anmeldenummer: PCT/EP2003/014292
(87) Internationale Veröffentlichungsnummer: WO 2004/056753

(56) Entgegenhaltungen:
- EP-A- 0 671 384
- EP-A- 0 985 659
- DE-A- 3 942 371
- US-A- 5 011 968
- US-A- 5 235 089

## Beschreibung

Die vorliegende Erfindung betrifft ein Verfahren zur Herstellung von 3-Cyano-3,5,5-trimethylcyclohexanon (Isophoronnitril) durch Umsetzung von Isophoron mit Cyanwasserstoff in Gegenwart einer Base als Katalysator, wobei vor einer an die Umsetzung anschließenden Destillation eine Neutralisation der als Katalysator eingesetzten Base erfolgt, sowie die Verwendung spezieller Sulfonsäuren zur Neutralisation einer als Katalysator eingesetzten Base in einem Verfahren zur Herstellung von Isophoronnitril.

3-Cyano-3,5,5-trimetbylcyclohexanon (Isophoronnitril) ist ein industriell bedeutendes Zwischenprodukt. Durch aminierende Hydrierung wird Isophoronnitril zu 1-Amino-3-aminomethyl-3,5,5-trimethylcyclohexan (Isophorondiamin) umgesetzt, das zur Herstellung von Epoxidharzen als Vernetzungsagens sowie als Spezialmonomer bei der Polyurethanherstellung eingesetzt wird.

Im allgemeinen wird Isophoronnitril basenkatalysiert durch Addition von Cyanwasserstoff an Isophoron bei erhöhter Temperatur hergestellt. Vor einer anschließenden destillativen Aufarbeitung des Isophoronnitril-Rohproduktes ist es vorteilhaft, die als Katalysator eingesetzte Base zu neutralisieren, um Zersetzungs- bzw. Rückspaltungsreaktionen des Isophoronnitrils und eine damit verbundene Ausbeuteverminderung zu vermeiden.

So betrifft DE-A 39 42 371 ein Verfahren zur Herstellung von Isophoronnitril durch Umsetzung von Isophoron mit Cyanwasserstoff in Gegenwart von Lithiumhydroxid als Katalysator, wobei eine Neutralisation des eingesetzten Lithiumhydroxids durch Phosphorsäure oder p-Toluolsulfonsäure erfolgt. Bei Einsatz dieser Verbindungen zur Neutralisation der als Katalysator eingesetzten Base werden kristalline Niederschläge gebildet, die zu erheblichen Problemen, bedingt durch Ablagerungen im Neutralisationsreaktor und in der nachgeschalteten Destillationskolonne, fuhren.

DE-A 1 085 871 betrifft ebenfalls ein Verfahren zur Herstellung von alicyclischen Cyanketonen, insbesondere von Isophoronnitril, durch Umsetzung von Cyanwasserstoff mit Isophoron in Gegenwart eines stark alkalischen Cyanidionen bildenden Katalysators. Als Neutralisationsmittel wird gemäß der Beispiele Phosphorsäure eingesetzt, was, wie bereits vorstehend erwähnt, zum Auftreten von kristallinen Niederschlägen führt.

US 5,183,915 betrifft ebenfalls ein Verfahren zur Herstellung von Isophoronnitril durch Umsetzung von Isophoron mit Cyanwasserstoff bei erhöhten Temperaturen und Drucken in Anwesenheit eines quartären Ammoniumcyanids oder quartären Phosphoniumcyanids als Katalysator.

DE-A 1 240 854 betrifft wiederum ein Verfahren zur Herstellung von Isophoronnitril durch Umsetzung von Isophoron mit Cyanwasserstoff in Gegenwart stark alkalischer, Cyanidionen bildender Katalysatoren. Gemäß den Beispielen wird als Neutralisationsmittel im Anschluß an die Reaktion Salpetersäure zugegeben. Auch bei der Zugabe von Salpetersäure bilden sich kristalline Niederschläge, die zu erheblichen Problemen bedingt durch Ablagerungen im Neutralisationsreaktor oder in der nachgeschalteten Destillationskolonne führen.

US 5,235,089 betrifft ein Verfahren zur Herstellung von Isophoronnitril durch Umsetzung von Isophoron mit Cyanwasserstoff in Gegenwart von Lithiumhydroxid oder Lithiumcyanid als Katalysator. Um das Reaktionsgemisch anzusäuern, kann eine polyacide Säure zugegeben werden. Im Anschluß daran erfolgt eine Filtration, um das ausgefallene Lithiumsalz der eingesetzten Säure aus dem Reaktionsgemisch zu entfernen. Geeignete polyacide Säuren sind Maleinsäure, Oxalsäure, Schwefelsäure und Phosphorsäure. Gemäß der Beschreibung in US 5,235,089 werden bei Einsatz von Maleinsäure kristalline Niederschläge des entsprechenden Dilithiumsalzes erhalten und bei Einsatz von Phosphorsäure schlecht filterbare feine Niederschläge in Form von LiH₂PO₄.

Die bei der Neutralisation gemäß dem Stand der Technik entstehenden Niederschläge können zu erheblichen Problemen, bedingt durch Ablagerungen im Neutralisationsreaktor und in der nachgeschalteten Destillationskolonne, führen. Beispielsweise werden aufgrund dieser Niederschläge häufige Reinigungsoperationen notwendig, was ökonomisch ungünstige Anlagenstillstandszeiten nach sich zieht. Des weiteren werden aufgrund der Ablagerungen reduzierte Destillationsausbeuten und erhöhte Rückstandsmengen verursacht, welche aufwendig entsorgt werden müssen.

Aufgabe der vorliegenden Erfindung ist daher die Bereitstellung eines Verfahrens zur Herstellung von Isophoronnitril durch Umsetzung von Isophoron mit Cyanwasserstoff in Gegenwart einer Base als Katalysator, worin die Neutralisation der Base vor einer Destillation des Isophoronnitril-Rohproduktes so erfolgte, daß keine sedimentierenden Niederschläge auftreten. Dadurch können die Verfahrenskosten bei der Herstellung von reinem Isophoronnitril erheblich gesenkt werden.

Diese Aufgabe wird gelöst durch ein Verfahren zur Herstellung von 3-Cyano-3,5,5-trimethylcyclohexanon (Isophoronnitril) durch Umsetzung von Isophoron mit Cyanwasserstoff in Gegenwart einer Base als Katalysator, ausgewählt aus Alkali- und Erdalkalicyaniden, Alkali- und Erdalkalihydroxiden, Alkali- und Erdalkalioxiden und Alkali- und Erdalkalialkoholaten, wobei ein Isophoronnitril-Rohprodukt erhalten wird, und anschließende Destillation des Isophoronnitril-Rohprodukts.

Das erfindungsgemäße Verfahren ist dann dadurch gekennzeichnet, daß vor der Destillation mindestens eine Sulfonsäure ausgewählt aus der Gruppe bestehend aus Methansulfonsäure, Naphthalinsulfonsäuren, alkylsubstituierten Naphthalinsulfonsäuren und alkylsubstituierten Benzolsulfonsäuren mit einem Alkylrest mit ≥ 4 Kohlenstoffatomen zugegeben wird.

Geeignete Sulfonsäuren sind somit Methansulfonsäure, Naphthalin- oder alkylsubstituierte Naphthalinsulfonsäuren und alkylsubstituierte Benzolsulfonsäuren mit einem Alkylrest mit ≥ 4 Kohlenstoffatomen, bevorzugt 6 bis 14 Kohlenstoffatomen. Bevorzugt ist C₁₂-Alkylbenzolsulfonsäure.

Die genannten Sulfonsäuren dienen als Neutralisationsmittel zur Neutralisation der als Katalysator eingesetzten Base, um Zersetzungs- und/oder Rückspaltungsreaktionen des Isophoronnitrils zu vermeiden. Bei Einsatz der gemäß der vorliegenden Erfindung aufgeführten Sulfonsäuren als Neutralisationsmittel wird im Gegensatz zum Stand der Technik kein Niederschlag gebildet. Dadurch kann die Aufarbeitung des Isophoronnitril-Rohprodukts ohne zusätzliche Aufwendungen wie Filtration durchgeführt werden.

Die erfindungsgemäß eingesetzten Sulfonsäuren werden in allgemeinen in Form ihrer hochkonzentrierten wässrigen Lösung, z.B. ≥ 30 Gew.-% bis 80 Gew.-% der erfindungsgemäß eingesetzten Säuren in Wasser oder pur eingesetzt, z.B. bei Einsatz von Methansulfonsäure oder Dodecylbenzolsulfonsäure.

Die genannten Sulfonsäuren können allein oder im Gemisch mit weiteren Säuren zur Neutralisation eingesetzt werden. Bevorzugt werden die genannten Sulfonsäuren jeweils allein eingesetzt. Es ist auch möglich, eine oder mehrere der genannten Sulfonsäuren im Gemisch mit anorganischen mineralischen Säuren, z.B. Phosphorsäure oder Schwefelsäure einzusetzen, jedoch lediglich mit einem Mengenanteil an anorganischer mineralischer Säure, welcher keine Salzausfällungen verursacht. Der genaue Mengenanteil ist dabei von der als Katalysator eingesetzten Base sowie von der eingesetzten Sulfonsäure abhängig.

In einer bevorzugten Ausführungsform wird ein Gemisch aus 60 bis 95 Gew.-%, bevorzugt 70 bis 80 Gew.-% einer alkyl substituierten Naphthalinsulfonsäure, 3 bis 39 Gew.-%, bevorzugt 5 bis 15 Gew.-% Schwefelsäure und 0 bis 20 Gew.-%, bevorzugt 0,5 bis 15 Gew.-% Wasser eingesetzt.

Geeignete Katalysatoren sind Basen, ausgewählt aus der Gruppe bestehend aus Alkali- und Erdalkalialkoholaten, -oxiden, -hydroxiden und -cyaniden. Geeignete Katalysatoren sind beispielsweise Natriumethylat, Kaliumbutylat, Lithiumethylat, Magnesiumethylat, Natrium-, Kalium-, Lithium-, Magnesiummethylat, Natriumoxid, Kaliumhydroxid, Calciumoxid, Bariumhydroxid, Strontiumhydroxid, Natriumcyanid, Kaliumcyanid, Lithiumcyanid, Bariumcyanid, Magnesiumcyanid und Calciumcyanid. Ganz besonders bevorzugt sind Natrium-, Kalzium-, Magnesium-, Calciumoxid, Natrium-, Kalium-, Magnesium-, Calciumcyanid und Natriummethylat.

Die als Katalysator eingesetzte Base wird im allgemeinen in einer Menge von 0,01 bis 20 Gew.-%, bezogen auf das eingesetzte Isophoron, bevorzugt 0,05 bis 5 Gew.-%, besonders bevorzugt 0,1 bis 1 Gew.-% eingesetzt.

Das Molverhältnis von Isophoron zu Cyanwasserstoff beträgt in dem erfindungsgemäßen Verfahren im allgemeinen 0,6 zu 1 bis 7 zu 1, bevorzugt 1 zu 1 bis 3 zu 1, besonders bevorzugt 1,3 zu 1 bis 2,5 zu 1. Wird ein Überschuß Isophoron in dem erfindungsgemäßen Verfahren eingesetzt, so kann dieses gleichzeitig als Lösungsmittel dienen.

Im allgemeinen wird das erfindungsgemäße Verfahren in Abwesenheit von Lösungsmitteln durchgeführt, es sei denn ein Überschuß Isophoron dient als Lösungsmittel. Es ist jedoch auch möglich, das erfindungsgemäße Verfahren in einem Lösungsmittel durchzuführen. Geeignete Lösungsmittel sind Dimethylformamid, N,N-Dimethylacetamid, N-Methyl-2-pyrrolidon, Glycole oder Glycolether.

Das erfindungsgemäße Verfahren wird im allgemeinen bei Temperaturen von 80 bis 220 C, bevorzugt von 120 bis 200°C, besonders bevorzugt von 150 bis 200°C durchgeführt. Der Druck beträgt in dem erfindungsgemäßen Verfahren im allgemeinen 1 bis 5 bar, bevorzugt 1 bis 3 bar.

Das Produkt wird nach dem erfindungsgemäßen Verfahren in sehr hoher Reinheit erhalten.

Die zur Neutralisation der als Katalysator eingesetzten Base eingesetzte Sulfonsäure wird in einer Menge von im allgemeinen 0,5 bis 2, bevorzugt 0,7 bis 1,3, besonders bevorzugt 0,9 bis 1,1, ganz besonders bevorzugt 1 Säureäquivalent(en), bezogen auf 1 Basenäquivalent der als Katalysator eingesetzten Base, eingesetzt.

Die erfindungsgemäß zugegebene Sulfonsäure wird vor der Destillation des bei der erfindungsgemäßen Umsetzung erhaltenen Isophoronnitril-Umsetzungsprodukts zugegeben. Bevorzugt erfolgt die Zugabe nach dem Ende der Umsetzung des Isophorons mit Cyanwasserstoff.

Im Anschluß an die Zugabe der erfindungsgemäß eingesetzten Sulfonsäure erfolgt die Destillation des Isophoronnitril-Rohprodukts. Im allgemeinen wird eine fraktionierte Destillation im Vakuum durchgeführt.

In einer bevorzugten Ausführungsform der vorliegenden Erfindung umfaßt das erfindungsgemäße Verfahren folgende Schritte:
a) Isophoronnitril-Synthese durch Umsetzung von Isophoron mit Cyanwasserstoff in Gegenwart einer Base als Katalysator, ausgewählt aus Alkali- und Erdalkalicyaniden, Alkali- und Erdalkalihydroxiden, Alkali- und Erdalkalioxiden und Alkali- und Erdalkalialkoholaten, wobei ein Isophoronnitril-Rohprodukt erhalten wird,
b) Neutralisation der in Schritt a) erhaltenen Reaktionsmischung mit einer Sulfonsäure ausgewählt aus der Gruppe bestehend aus Methansulfonsäure, Naphthalinsulfonsäuren, alkylsubstituierten Naphthalinsulfonsäuren und alkylsubstituierten Benxolsulfonsäuren mit einem Alkylrest mit ≥ 4 Kohlenstoffatomen
c) Destillation der im Schritt b) erhaltenen Reaktionsmischung.

Bevorzugte Ausführungsformen der Schritte a), b), und c) und der darin eingesetzten Komponenten wurden bereits vorstehend erwähnt

Es ist mögliche die Isophoronnitril-Synthese, die anschließende Neutralisation und die destillative Aufarbeitung jeweils für sich kontinuierlich, semi-kontinuierlich oder in Batch-Fahrweise durchzuführen. Bevorzugt wird die Isophoronnitril-Synthese (Umsetzung) selbst kontinuierlich oder semi-kontnnuierlich, die Neutralisation (Zugabe der Sulfonsäure oder Carbonsäure) kontinuierlich oder semi-kontinuierlich und die anschließende Destillation (destillative Aufarbeitung) kontinuierlich oder semi-kontinuierlich durchgeführt. Besonders bevorzugt werden die Isophoronnitril-Synthese, die Neutralisation und die anschließende Destillation kontinuierlich durchgeführt.

Die einzelnen Verfahrensschritte können in getrennten Reaktoren durchgeführt werden. Geeignete Reaktoren sind dem Fachmann bekannt. In einer Ausführungsform des erfindungsgemäßen Verfahrens erfolgen sowohl die Isophoronnitril-Synthese als auch die anschließende Neutralisation in der Batch-Fahrweise. In dieser Ausführungsform werden die Isophoronnitril-Synthese und die anschließenden Neutralisation bevorzugt in demselben Reaktor durchgeführt. In den weiteren möglichen Ausführungsformen ist eine Durchführung der einzelnen Verfahrensschritte in getrennten Reaktoren bevorzugt. Die anschließende Destillation erfolgt im allgemeinen mit einer Rektifikationskolonne. Es kommen grundsätzlich alle geeigneten Reaktortypen in Frage.

Ein weiterer Gegenstand der vorliegenden Anmeldung ist die Verwendung einer Sulfonsäure ausgewählt aus der Gruppe bestehend aus Methansulfonsäure, Naphthalinsulfonsäuren, alkylsubstituierten Naphthalhisulfonsäuren und alkylsubstituierten Benzolsulfonsäuren mit einem Alkylrest mit ≥ 4 Kohlenstoffatomen als Neutralisationsmittel bei der Destillation eines Isophoronnitril-Rohprodukts, das durch Umsetzung von Isophoron mit Cyanwasserstoff in Anwesenheit einer Base ausgewählt aus Alkali- und Erdalkalicyaniden, Alkali- und Erdalkalihydroxideza, Alkali- und Erdalkalioxiden und Alkali- und Erdalkalialkoholaten als Katalysator erhalten wurde, zur Vermeidung von Niederschlägen bei der Neutralisation.

Bevorzugte Sulfonsäuren wurden vorstehend erwähnt.

Unter Neutralisation bzw. Neutralisationsmittel ist dabei die Neutralisation der als Katalysator eingesetzten Base zu verstehen, die bei der Reinigung des Isophoronnitril-Rohprodukts durch Destillation Zersetzung- und/oder Rückspaltungsreaktionen des als Wertprodukt erhaltenen Isophoronnitrils verursachen kann, bzw. die zur Neutralisation eingesetzte(n) Verbindung(en).

Die nachfolgenden Beispiele erläutern die Erfindung zusätzlich.

### Beispiele

### Beispiel 1: (Vergleichsbeispiel)

Es wurden 500 g einer Isophoronnitril-Rohsyntheselösung mit 54,8 Gew.-% Isophoronnitril (= 274 g), 41,1 % Isophoron (= 206 g) und 0,3 % Natriumcyanid (= 1,5 g = 0,03 mol) in einer batch-Destillationsapparatur vorgelegt. Die Neutralisation der Base erfolgte bei 50 °C mit 3,9 g 75%iger wäßriger Phosphorsäure.

Es fiel augenblicklich ein grob kristalliner Niederschlag aus. Es wurde bei 50 mbar ca. 2 Stunden fraktionierend bis zu einer Sumpftemperatur von ca. 200 °C destilliert.

| | | |
|---|---|---|
| Ausbeute: | Isophoron: 195 g | Isophoronnitril: 245 g |
| | Destillationsrückstand: 48 g (kristallin) | |

### Beispiel 2: (Vergleichsbeispiel)

Isophoronnitril-Rohlösung wie in Beispiel 1. Anstelle der Phosphorsäure wurden 4,8 g Toluolsulfonsäure zur Neutralisation eingesetzt.

Es fiel ein kristalliner Niederschlag aus, allerdings feiner als in Beispiel 1. Die Destillationsbedingungen waren analog zu Beispiel 1.

| | | |
|---|---|---|
| Ausbeute: | Isophoron: 198 g | Isophoronnitril: 253 g |
| | Rückstand: 44 g (fest) | |

### Beispiel 3: (erfindungsgemäß)

Isophoronnitril-Rohlösung wie in Beispiel 1. Zur Neutralisation wurden 4,1 g 70%ige wäßrige Methansulfonsäure verwendet.

Es trat kein kristalliner Niederschlag auf. Die Destillationsbedingungen waren analog zu Beispiel 1.

| | | |
|---|---|---|
| Ausbeute: | Isophoron: 202 g | Isophoronnitril: 259 g |
| | Rückstand: 19 g (Das Öl erstarrte erst unter 50 °C glasartig) | |

### Beispiel 4: (erfindungsgemäß)

Isophoronnitril-Rohlösung wie in Beispiel 1. Zur Neutralisation wurden 9,1 g Nekal SBC^{®} mit folgender Zusammensetzung eingesetzt: 75 Gew.-% Düsobutylnaphthalinsulfonsäure, 10 Gew.-% Schwefelsäure und 15 Gew.-% Wasser.

Es trat weder kristalliner Niederschlag noch eine Trübung auf. Die Destillationsbedingungen waren analog zu Beispiel 1.

| | | |
|---|---|---|
| Ausbeute: | Isophoron: 201 g | Isophoronnitril: 265 g |
| | Rückstand: 15 g (bleibt auch bei Raumtemperatur ein Öl) | |

### Beispiel 5: (erfindungsgemäß)

Isophoronnitril-Rohlösung wie in Beispiel 1. Zur Neutralisation wurden 9,7 g einer Dodecyl-benzolsulfonsäure (LAS (lineare Alkylbenzolsulfonsäure, Lutensit^{®} ALBS) - mit C₁₂-Alkyl im Durchschnitt) eingesetzt.

Es trat keinerlei Niederschlag oder Trübung auf. Die Destillationsbedingungen waren analog zu Beispiel 1.

| | | |
|---|---|---|
| Ausbeute: | Isophoron: 200 g | Isophoronnitril: 263 g |
| | Rückstand: 18 g (Öl) | |

### Beispiel 6: (Vergleichsbeispiel)

Es wurden 500 g eines Roh-Isophoronnitril folgender Zusammensetzung verwendet: 41,7 % Isophoron (= 210 g), 55,3 % Isophoronnitril (= 277 g), 1,7 g Calciumoxid (= 0,03 mol). Zur Neutralisation wurden 3,9 g 75%ige wäßrige Phosphorsäure eingesetzt. Es fiel augenblicklich ein grob kristalliner Niederschlag aus. Die Destillationsbedingungen waren analog zu Beispiel 1.

| | | |
|---|---|---|
| Ausbeute: | Isophoron: 198 g | Isophoronnitril: 248 g |
| | Rückstand: 38 g (fest) | |

### Beispiel 7: (erfindungsgemäß)

Die Durchführung erfolgte analog zu Beispiel 6. Zur Neutralisation wurden 4,1 g 70%ige wäßrige Methansulfonsäure verwendet.

Es fiel kein Niederschlag aus. Die Destillationsbedingungen waren analog zu Beispiel 1.

| | | |
|---|---|---|
| Ausbeute: | Isophoron: 203 g | Isophoronnitril: 264 g |
| | Rückstand: 18 g (erstarrt erst unter 50 °C glasartig) | |

### Beispiel 8: (Vergleichsbeispiel)

Isophoronnitril-Rohlösung wie in Beispiel 1. Zur Neutralisation wurden 4,3g 2-Ethylhexansäure eingesetzt.

Es trat lediglich eine schwache Trübung auf. Die Destillationsbedingungen waren analog zu Beispiel 1.

| | | |
|---|---|---|
| Ausbeute: | Isophoron: 201 g | Isophoronnitril: 259 g |
| | Rückstand: 25 g (viskoses Öl) | |

## Patentansprüche

1. Verfahren zur Herstellung von 3-Cyano-3,5,5-trimethylcyclohexanon (Isophoronnitril) durch Umsetzung von Isophoron mit Cyanwasserstoff in Gegenwart einer Base als Katalysator, ausgewählt aus Alkali- und Erdalkalicyaniden, Alkali- und Erdalkalihydroxiden, Alkali- und Erdalkalioxiden und Alkali- und Erdalkalialkoholaten, wobei ein Isophoronnitril-Rohprodukt erhalten wird, und anschließende Destillation des Isophoronnitril-Rohprodukts, **dadurch gekennzeichnet, daß** vor der Destillation mindestens eine Sulfonsäure ausgewählt aus der Gruppe bestehend aus Methansulfonsäure, Naphthalinsulfonsäuren, alkylsubstituierten Naphthalinsulfonsäuren und alkylsubstituierten Benzolsulfonsäuren mit einem Alkylrest mit ≥ 4 Kohlenstoffatomen zugegeben wird.

2. Verfahren nach Anspruch 1, **dadurch gekennzeichnet, daß** die Sulfonsäure in einer Menge von 1 Säureäquivalent, bezogen auf 1 Basenäquivalent der als Katalysator eingesetzten Base, eingesetzt wird.

3. Verfahren nach Anspruch 1 oder 2, **dadurch gekennzeichnet, daß** die als Katalysator eingesetzte Base in einer Menge von 0,01 bis 20 Gew.-%, bezogen auf das eingesetzte Isophoron, eingesetzt wird.

4. Verfahren nach einem der Ansprüche 1 bis 3, **dadurch gekennzeichnet, daß** die Umsetzung bei Temperaturen von 80 bis 220°C, bevorzugt von 120 bis 200°C, besonders bevorzugt 150 bis 200°C, durchgeführt wird.

5. Verfahren nach einem der Ansprüche 1 bis 4, **dadurch gekennzeichnet, daß** die Umsetzung bei einem Druck von 1 bis 5 bar, bevorzugt 1 bis 3 bar durchgeführt wird.

6. Verfahren nach einem der Ansprüche 1 bis 5, **dadurch gekennzeichnet, daß** die Destillation in einer Rektifikationskolonne durchgeführt wird.

7. Verfahren nach einem der Ansprüche 1 bis 6, **dadurch gekennzeichnet, daß** die Umsetzung, die Zugabe der Sulfonsäure und die anschließende Destillation kontinuierlich durchgeführt werden.

8. Verfahren nach einem der Ansprüche 1 bis 7, umfassend folgende Schritte:
a) Isophoronnitril-Synthese durch Umsetzung von Isophoron mit Cyanwasserstoff in Gegenwart einer Base als Katalysator, ausgewählt aus Alkali- und Erdalkalicyaniden, Alkali- und Erdalkalihydroxiden, Alkali- und Erdalkalioxiden und Alkali- und Erdalkalialkoholaten, wobei ein Isophoronnitril-Rohprodukt erhalten wird,
b) Neutralisation der in Schritt a) erhaltenen Reaktionsmischung mit einer Sulfonsäure ausgewählt aus der Gruppe bestehend aus Methansulfonsäure, Naphthalinsulfonsäuren, alkylsubstituierten Naphthalinsulfonsäuren und alkylsubstituierten Benzolsulfonsäuren mit einem Alkylrest mit ≥ 4 Kohlenstoffatomen
c) Destillation der im Schritt b) erhaltenen Reaktionsmischung.

9. Verwendung einer Sulfonsäure ausgewählt aus der Gruppe bestehend aus Methansulfonsäure, Naphthalinsulfonsäuren, alkylsubstituierten Naphthalinsulfonsäuren und alkylsubstituierten Benzolsulfonsäuren mit einem Alkylrest mit ≥ 4 Kohlenstoffatomen als Neutralisationsmittel vor der Destillation eines Isophoronnitril-Rohprodukts, das durch Umsetzung von Isophoron mit Cyanwasserstoff in Anwesenheit einer Base ausgewählt aus Alkali- und Erdalkalicyaniden, Alkali- und Erdalkalihydroxiden, Alkali- und Erdalkalioxiden und Alkali- und Erdalkalialkoholaten als Katalysator erhalten wurde, zur Vermeidung von Niederschlägen bei der Neutralisation der als Katalysator eingesetzten Base mit einer Säure.

10. Verfahren nach einem der Ansprüche 1 bis 8, **dadurch gekennzeichnet, dass** die Sulfonsäure Düsobutylnaphthalinsulfonsäuren oder Dodecyl-Benzolsulfonsäure ist.

11. Verwendung nach Anspruch 9, **dadurch gekennzeichnet, dass** die Sulfonsäure Düsobutylnaphthalinsulfonsäure oder Dodecyl-Benzol-Sulfonsäure ist.

## Claims

1. A process for preparing 3-cyano-3,5,5-trimethylcyclohexanone (isophoronenitrile) by reacting isophorone with hydrogen cyanide in the presence of a base as a catalyst, selected from alkali metal and alkaline earth metal cyanides, alkali metal and alkaline earth metal hydroxides, alkali metal and alkaline earth metal oxides and alkali metal and alkaline earth metal alkoxides, to obtain a crude isophoronenitrile product, and subsequently distilling the crude isophoronenitrile product, which comprises adding, before the distillation, at least one sulfonic acid selected from the group consisting of methanesulfonic acid, naphthalenesulfonic acids, alkyl-substituted naphthalenesulfonic acids and alkyl-substituted benzenesulfonic acids having an alkyl radical having ≥ 4 carbon atoms.

2. The process according to claim 1, wherein the sulfonic acid is used in an amount of 1 acid equivalent, based on 1 base equivalent of the base used as the catalyst.

3. The process according to claim 1 or 2, wherein the base used as the catalyst is used in an amount of from 0.01 to 20% by weight, based on the isophorone used.

4. The process according to any of claims 1 to 3, wherein the reaction is carried out at temperatures of from 80 to 220°C, preferably from 120 to 200°C, more preferably from 150 to 200°C.

5. The process according to any of claims 1 to 4, wherein the reaction is carried out at a pressure of from 1 to 5 bar, preferably from 1 to 3 bar.

6. The process according to any of claims 1 to 5, wherein the distillation is carried out in a rectification column.

7. The process according to any of claims 1 to 6, wherein the reaction, the addition of the sulfonic acid and the subsequent distillation are carried out continuously.

8. The process according to any of claims 1 to 7, comprising the following steps:
a) isophoronenitrile synthesis by reaction of isophorone with hydrogen cyanide in the presence of a base as a catalyst, selected from alkali metal and alkaline earth metal cyanides, alkali metal and alkaline earth metal hydroxides, alkali metal and alkaline earth metal oxides and alkali metal and alkaline earth metal alkoxides, to obtain a crude isophoronenitrile product,
b) neutralization of the reaction mixture obtained in step a) with a sulfonic acid selected from the group consisting of methanesulfonic acid, naphthalenesulfonic acids, alkyl-substituted naphthalenesulfonic acids and alkyl-substituted benzenesulfonic acids having an alkyl radical having ≥ 4 carbon atoms.
c) distillation of the reaction mixture obtained in step b).

9. The use of a sulfonic acid selected from the group consisting of methanesulfonic acid, naphthalenesulfonic acids, alkyl-substituted naphthalenesulfonic acids and alkyl-substituted benzenesulfonic acids having an alkyl radical having ≥ 4 carbon atoms as a neutralizing agent before the distillation of a crude isophoronenitrile product which has been obtained by reacting isophorone with hydrogen cyanide in the presence of a base selected from alkali metal and alkaline earth metal cyanides, alkali metal and alkaline earth metal hydroxides, alkali metal and alkaline earth metal oxides and alkali metal and alkaline earth metal alkoxides as a catalyst, in order to avoid precipitates in the neutralization of the base used as the catalyst with an acid.

10. The process according to any of claims 1 to 8, wherein the sulfonic acid is diisobutylnaphthalenesulfonic acids or dodecylbenzenesulfonic acid.

11. The use according to claim 9, wherein the sulfonic acid is diisobutylnaphthalenesulfonic acid or dodecylbenzenesulfonic acid.

## Revendications

1. Procédé pour la préparation de 3-cyano-3,5,5-triméthylcyclohexanone (isophorone-nitrile) par transformation d'isophorone avec du cyanure d'hydrogène en présence d'une base comme catalyseur, choisie parmi les cyanures de métal alcalin et alcalino-terreux, les hydroxydes de métal alcalin et alcalino-terreux, les oxydes de métal alcalin et alcalino-terreux et les alcoolates de métal alcalin et alcalino-terreux, où on obtient un produit brut d'isophoronenitrile, et par distillation consécutive du produit brut d'isophorone-nitrile, **caractérisé en ce qu'**on ajoute, avant la distillation, au moins un acide sulfonique choisi dans le groupe constitué par l'acide méthanesulfonique, les acides naphtalènesulfoniques, les acides naphtalènesulfoniques substitués par alkyle et les acides benzènesulfoniques substitués par alkyle avec un radical alkyle comprenant ≥ 4 atomes de carbone.

2. Procédé selon la revendication 1, **caractérisé en ce que** l'acide sulfonique est ajouté en une quantité de 1 équivalent en acide par rapport à 1 équivalent en base de la base utilisée comme catalyseur.

3. Procédé selon la revendication 1 ou 2, **caractérisé en ce que** la base ajoutée comme catalyseur est utilisée en une quantité de 0,01 à 20% en poids par rapport à l'isophorone utilisée.

4. Procédé selon l'une quelconque des revendications 1 à 3, **caractérisé en ce que** la transformation est réalisée à des températures de 80 à 220°C, de préférence de 120 à 200°C, de manière particulièrement préférée de 150 à 200°C.

5. Procédé selon l'une quelconque des revendications 1 à 4, **caractérisé en ce que** la transformation est réalisée à une pression de 1 à 5 bars, de préférence de 1 à 3 bars.

6. Procédé selon l'une quelconque des revendications 1 à 5, **caractérisé en ce que** la distillation est réalisée dans une colonne de rectification.

7. Procédé selon l'une quelconque des revendications 1 à 6, **caractérisé en ce que** la transformation, l'addition de l'acide sulfonique et la distillation consécutive sont réalisées en continu.

8. Procédé selon l'une quelconque des revendications 1 à 7, comprenant les étapes suivantes :
a) synthèse d'isophoronenitrile par transformation d'isophorone avec du cyanure d'hydrogène en présence d'une base comme catalyseur, choisie parmi les cyanures de métal alcalin et alcalino-terreux, les hydroxydes de métal alcalin et alcalino-terreux, les oxydes de métal alcalin et alcalino-terreux et les alcoolates de métal alcalin et alcalino-terreux, où on obtient un produit brut d'isophoronenitrile,
b) neutralisation du mélange réactionnel obtenu dans l'étape a) avec un acide sulfonique choisi dans le groupe constitué par l'acide méthanesulfonique, les acides naphtalènesulfoniques, les acides naphtalènesulfoniques substitués par alkyle et les acides benzènesulfoniques substitués par alkyle avec un radical alkyle comprenant ≥ 4 atomes de carbone, et
c) distillation du mélange réactionnel obtenu dans l'étape b).

9. Utilisation d'un acide sulfonique choisi dans le groupe constitué par l'acide méthanesulfonique, les acides naphtalènesulfoniques, les acides naphtalènesulfoniques substitués par alkyle et les acides benzènesulfoniques substitués par alkyle avec un radical alkyle comprenant ≥ 4 atomes de carbone comme agent de neutralisation avant la distillation d'un produit brut de type isophoronenitrile, qui a été obtenu par transformation d'isophorone avec du cyanure d'hydrogène en présence d'une base choisie parmi les cyanures de métal alcalin et alcalino-terreux, les hydroxydes de métal alcalin et alcalino-terreux, les oxydes de métal alcalin et alcalino-terreux et les alcoolates de métal alcalin et alcalino-terreux comme catalyseur pour éviter des précipités lors de la neutralisation de la base utilisée comme catalyseur avec un acide.

10. Procédé selon l'une quelconque des revendications 1 à 8, **caractérisé en ce que** l'acide sulfonique réside dans les acides diisobutylnaphtalènesulfoniques ou l'acide dodécylbenzènesulfonique.

11. Utilisation selon la revendication 9, **caractérisée en ce que** l'acide sulfonique est l'acide diisobutylnaphtalènesulfonique ou l'acide dodécylbenzènesulfonique.
